Europäisches Patentamt

European Patent Office

Office européen des brevets

(11) Publication number: **0 375 566 B1**

(12) **EUROPEAN PATENT SPECIFICATION**

(45) Date of publication of patent specification: **20.10.93** (51) Int. Cl.5: **G01N 33/48**, B01D 43/00

(21) Application number: **89403644.1**

(22) Date of filing: **22.12.89**

The file contains technical information submitted after the application was filed and not included in this specification

(54) **Blood separating agent.**

(30) Priority: **22.12.88 JP 324474/88**

(43) Date of publication of application:
**27.06.90 Bulletin  90/26**

(45) Publication of the grant of the patent:
**20.10.93 Bulletin  93/42**

(84) Designated Contracting States:
**BE CH DE ES FR GB IT LI NL SE**

(56) References cited:
**EP-A- 0 039 898**
**JP-A-62 197 765**
**US-A- 4 310 430**
**US-A- 4 569 764**

(73) Proprietor: **TERUMO KABUSHIKI KAISHA**
**No. 44-1, Hatagaya 2-chome,**
**Shibuya-ku**
**Tokyo 151(JP)**

Proprietor: **MITSUBISHI KASEI CORPORATION**
**5-2, Marunouchi 2-chome**
**Chiyoda-ku**
**Tokyo 100(JP)**

(72) Inventor: **Tachikawa, Koichi, c/o Terumo K.K.**

**1727-1, Tsuiji-shinkyo,**
**Showa-cho**
**Nakakoma-gun, Yamanashi-ken(JP)**
Inventor: **Akaike, Yoshiaki, c/o Terumo K.K.**
**1727-1, Tsuiji-shinkyo,**
**Showa-cho**
**Nakakoma-gun, Yamanashi-ken(JP)**
Inventor: **Yamanouchi, Hideki**
**1438-21, Kanamori**
**Machida-shi Tokyo(JP)**
Inventor: **Mori, Tomoyuki**
**1-66-23, Hiroe 3-chome**
**Kurashiki-shi Okayama-ken(JP)**

(74) Representative: **Gillard, Marie-Louise et al**
**Cabinet Beau de Loménie**
**158, rue de l'Université**
**F-75340 Paris Cédex 07 (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

BACKGROUND OF THE INVENTION

Field of the Invention:

This invention relates to a blood separating agent. More particularly, this invention, in the separation of blood between a serum component and a clot component or between a blood plasma component and a blood corpuscle component by virtue of their difference in specific gravity, relates to a blood separating agent having a specific gravity of the degree of an intermediate between the two components thereby permitting formation of a septum between the two components and facilitating the operation of separating the two components and to a blood collecting tube having the blood separating agent encapsulated in a blind tube.

Description of the Prior Art:

For use in the operation of separating the blood between a blood clot and a blood serum or between blood corpuscles and a blood plasma, various separating agents have been heretofore introduced to the art. In the separating agents of this kind, the blood separating agent which is composed of a separated layers forming material having as its main component an $\alpha$-olefin-maleic diester copolymer possessing a viscosity in the range of 10 to 80 Pa.s (10,000 to 80,000 cP) (at 25°C), a specific gravity and viscosity adjusting agent such as silica, clay, and optionally a structure forming agent such as a dimethyl polysiloxane-polyoxyalkylene copolymer or Carbitol (a high-boiling ether-alcohol) has been renowned (USP 4,310,430). The blood separating agent of this composition, however, is substantially heterogeneous because it contains as a thixotropic agent in a dispersed state a fine inorganic powder insoluble in the separated layers forming material. This blood separating agent has a disadvantage that, under the conditions of protracted preservation or centrifugal separation, it induces phase separation between the separated layers forming material and the specific gravity and viscosity adjusting agent and suffers from degradation of the ability of separation due to changes in physical properties of gel. When this blood separating agent is used for the separation of blood serum, it does not fully manifest its separating function because blood corpuscles are liable to remain on the supernatant surface of the separating agent, namely in the separated blood serum, after the step of centrifugal separation. In the centrifugal separation of blood serum or blood plasma, the blood separating agent is still incapable of fully manifesting the separating function thereof because the separated blood portion, on being left standing in the state assumed in consequence of the separation, suffers a portion possessing a concentration gradient to migrate through the medium of the separating agent between the blood clot and the blood serum or between the blood corpuscles and the blood plasma often, if not always, to the extent of impairing the results of the analysis of blood serum or blood plasma.

For the purpose of solving this problem, a blood separating agent which comprises a separated layers forming material formed of a silicone, chlorinated polybutene, chlorinated polystyrene, a polyacrylic ester, a copolymer of an $\alpha$-olefin or styrene with an $\alpha$, $\beta$-unsaturated dicarboxylic ester and an organic gelling agent such as the condensate of sorbitol with benzaldehyde, a nitrohumic acid adduct of water-soluble protein, hydrated castor oil, or 12-hydroxystearic acid has been proposed (Japanese Patent Laid-Open SHO 62-(1987)-197,765). This blood separating agent is unstable in terms of the ability to retain shape of its own and is still deficient in stability of preservation. Moreover, this blood separating agent shows a conspicuous trend toward increasing the minimum shear stress required for conversion of the separating agent into the sol, degrading the floatability of the separating agent during the operation of centrifugal separation, lowering the function of the separation, and so on.

An object of this invention, therefore, is to provide a novel blood separating agent and a blood separating tube having the separating agent encapsulated in a blind tube.

Another object of this invention is to provide, for the separation of the blood between a blood serum component and a blood clot component or between a blood plasma component and a blood corpuscle component by virtue of the difference of specific gravity therebetween, a blood separating agent having an intermediate specific gravity between the two components thereby effecting formation of a septum between the two components and consequently facilitating the operation of separation of the two components and to provide a blood separating device produced by having this blood separating agent encapsulated in a blind tube.

## SUMMARY OF THE INVENTION

The objects described above are accomplished by a blood separating agent comprising (A) a separated layers forming material formed of a copolymer of an $\alpha$-olefin of 6 to 20 carbon atoms with a maleic diester in which the number of carbon atoms of the alkyl group is in the range of 1-4, and possessing a compositional ratio of 1.0 to 1.4 mols of the maleic diester per 1.0 mol of the $\alpha$-olefin in the copolymer and a viscosity in the range of 100 to 250 Pa.s (100,000 to 250,000 cP) at 25°C and (B) 0.12 to 0.30 part by weight, based on 100 parts by weight of said separated layers forming material, of an organic gelling agent formed of a condensate of sorbitol and benzaldehyde, and the blood separating agent possessing a specific gravity in the range of 1.028 to 1.050 (at 25°C).

The objects described above are also accomplished by a blood separating tube having the aforementioned blood separating agent encapsulated in a blind tube.

The blood separating agent of this invention is stable to withstand the impact of aging, incapable of being fluidified within the blood separating tube under the impact of transportation during the course of transportation of the tube, and inactive to the blood. In other words, the blood separating agent has no possibility of inducing such phenomena as adsorption and elution of the blood. It is not affected physically or chemically under the impact of sterilization with radiation such as $\gamma$ ray.

When the blood separating agent is used for the purpose of separating blood serum, no blood corpuscle is suffered to remain on the supernatant surface of the separating agent, namely in the blood serum separated, after the step of centrifugal separation. When the blood separating agent is used for the purpose of separating blood serum or blood plasma, even when the portion resulting from the centrifugal separation is left standing per se, migration of a portion possessing a concentration gradient between the blood clot and the blood serum or between the blood corpuscles and the blood plasma through the medium of the separating agent does not occur and, therefore, the analysis of blood components is allowed to yield accurate results.

## BRIEF DESCRIPTION OF THE DRAWINGS

Fig. 1 is a perspective view illustrating the blood separating agent of this invention used for the separation of blood serum as encapsulated in a blood collector tube, and

Fig. 2 is a sectional view illustrating the blood collection tube of Fig. 1 after centrifugal separation.

## EXPLANATION OF THE PREFERRED EMBODIMENT

Now, the present invention will be described in detail below.

The separated layers forming material for use in the blood separating agent of this invention is a copolymer of an $\alpha$-olefin of 6 to 20, preferably 10 to 16, carbon atoms and a maleic diester. This copolymer contains these components in a compositional ratio of 1.0 to 1.4 mols, of the maleic diester to 1.0 mol of the $\alpha$-olefin and possesses a viscosity in the range of 100 to 250 Pa.s (100,000 to 250,000 cP), preferably 120 to 200 Pa.s (120,000 to 200,000 cP) (at 25°C).

The $\alpha$-olefin of 6 to 20 carbon atoms for use in this invention is a hydrocarbon of linearity or a low branching coefficient possessing a carbon-to-carbon double bond at a terminal thereof. Specifically, the product of low polymerization of ethylene is used favorably and the products of low polymerization of propylene, isobutylene, etc. are also usable. The $\alpha$-olefins possessing carbon atoms in the specified range are obtained by means of distillation. For this invention, the $\alpha$-olefins of 6 to 20 carbon atoms may be used either singly or in the form of two or more members. An $\alpha$-olefin having not less than 20 carbon atoms is undesirable because the produced copolymer using this $\alpha$-olefin assumes a solid state at room temperature. An $\alpha$-olefin having not more than 6 carbon atoms is undesirable because the synthesis of the copolymer entails complicated operations such as the necessity for performing the reaction under application of pressure.

The alcohol as a raw material for the maleic diester is preferable to have 1 to 14 carbon atoms. The alcohol having not less than 14 carbon atoms is undesirable because the produced copolymer using this alcohol possesses an unduly high pour point and undergoes solidification. Besides, an increase in the number of carbon atoms of the alcohol proportionately adds to the difficulty with which the unreacted maleic diester is removed by distillation from the copolymerization mixture. Since alcohols having smaller numbers of carbon atoms are more preferable than those of larger numbers of carbon atoms, the alcohols of 1 to 8 carbon atoms are used favorably and methanol is used most preferably. The alcohols which are used advantageously herein include methanol, ethanol, propanol, isopropanol, butanol, isobutanol, sec-butanol,

3

hexanols, heptanols, and octanols, for example.

The production of the $\alpha$-olefin-maleic diester copolymer is accomplished by combining an $\alpha$-olefin and a maleic diester in respectively prescribed amounts and inducing them to react with each other at a temperature in the range of 60 to 200°C, preferably 70 to 180°C, in the presence of a radical polymerization initiator.

For the blood separating agent to acquire a desired quality, the compositional ratio of the $\alpha$-olefin to the maleic diester in the copolymer serving as the separated layers forming material is required to be 1.0 to 1.4 mols of the maleic diester to 1.0 mol of the $\alpha$-olefin.

It is likewise necessary that the copolymer of the $\alpha$-olefin and the maleic diester should possess a viscosity in the range of 100 to 250 Pa.s (100,000 to 250,000 cP) at 25°C. A viscosity of not more than 100 Pa.s (100,000 cP) is undesirable because the produced separating agent is deficient in stability of storage and is liable to migrate under the influence of gravity at elevated temperatures and retain its shape with difficulty. Where the separating agent is encapsulated in the bottom part of a vacuum blood collecting tube, for example, there ensues the possibility that the separating agent will migrate to the neighborhood of the rubber stopper keeping the mouth of the vacuum blood collecting tube tightly closed and, after the separation of blood, continue to remain on the blood serum or blood plasma component side or on the rubber stopper and render the collection of blood serum or blood plasma difficult or, at times, pollute the portion in question.

Further since the timing for the floatation of the blood separating agent during the course of the centrifugal separation is advanced and the timing for the formation of the septum of the separating agent is proportionately advanced, blood corpuscles are liable to remain on the supernatant surface of the separating agent and impair the sufficiency of the performance of separation when blood separating agent is used for the separation of blood serum.

If the viscosity exceeds 250 Pa.s (250,000 cP), there arises a disadvantage that the blood separating agent is too viscous to be easily handled and dispensed among blood collecting tubes and, therefore, is deficient in floatation of the blood separating agent and performance of separation.

Preferably, the viscosity of the copolymer is approximately in the range of 120,000 to 200,000 centipoises. The viscosity can be effectively adjusted by adjusting the polymerization degree or molecular weight of the copolymer.

From the standpoint of the physical properties of the blood separating agent produced by using the copolymer of the $\alpha$-olefin and the maleic diester as the separated layers forming material, the specific gravity of the copolymer is preferable to be in the range of 1.028 to 1.050 at 25°C. The specific gravity can be suitably adjusted by selecting the $\alpha$-olefin and the maleic diester with respect to the number of carbon atoms and the compositional ratio of the components of the copolymer.

The organic gelling agent to be used in the blood separating agent of the present invention is the condensate of sorbitol and an benzaldehyde. The condensates which are usable herein include dibenzylidene sorbitol, tribenzylidene sorbitol, and methyl-substituted dibenzylidene sorbitol, for example. Among other condensates mentioned above, dibenzylidene sorbitol proves to be particularly preferable.

The condensate is inactive to blood. When it is added in a small amount to the separated layers forming material, it enables the produced blood separating agent to manifest a behavior necessary for a gel.

The proper amount of addition of the organic gelling agent is in the range of 0.12 to 0.30 part by weight, preferably 0.14 to 0.30 part by weight, based on 100 parts by weight of the separated layers forming material mentioned above.

When the amount of addition of the organic gelling agent is unduly small, the blood separating agent during the course of blood separation manifests an insufficient strength and suffers the septum to fluidity and, therefore, fails to function sufficiently as a separating agent. Additionally the blood separating agent retains in its shape with difficulty and in deficient in stability of storage. On the other hand, the amount of addition of the organic gelling agent is unduly large, the fluidity becomes insufficient, so the floatability of the separating agent during centrifugation becomes difficult and fluidity of the separating agent decreases, and as a result, separation function decreases.

The blood separating agent of the present invention can easily be produced by heating the $\alpha$-olefin-maleic diester copolymer which is a separated layers forming material at a temperature in the range of 100° to 200°C, preferably 120° to 180°C, adding the organic gelling agent into it, and stirring the resultant mixture under heating until the mixture becomes homogeneous and transparent.

The blood separating agent of this invention is required to possess a specific gravity intermediate between the blood serum component and the blood clot or between the blood plasma and the blood corpuscles. The specific gravity of the blood separating agent, therefore, is in the range of 1.028 to 1.050, more preferably 1.030 to 1.045 at 25°C. In the case of the blood separating tube having the blood separating agent encapsulated in the bottom portion thereof, for example, the difference in specific gravity between the blood clot component or the blood corpuscle component and the blood separating agent is preferable to be as large as possible because the floatability of the separating agent during the course of the centrifugal separation increases in proportion as the difference in specific gravity increases. A specific gravity of less than 1.028 is undesirable because part of the separating agent of such a small specific gravity may possibly pass into the blood serum or the blood plasma component after the step of centrifugal separation.

The blood separating agent of this invention which comprises the specific $\alpha$-olefin-maleic diester copolymer and the specific amount of the organic gelling agent exhibits a viscosity in the range of 300 to 700 Pa.s (300,000 to 700,000 cP), preferably 400 to 700 Pa.s (400,000 to 700,000 cP) at 25°C (as measured by the method which will be described specifically hereinafter).

The blood separating agent of this invention, for the purpose of allowing the specific gravity and the viscosity thereof to be controlled to their respectively desired values, may further incorporate therein any of the well known finely divided inorganic powders such as silica, clay, and titanium dioxide, for example.

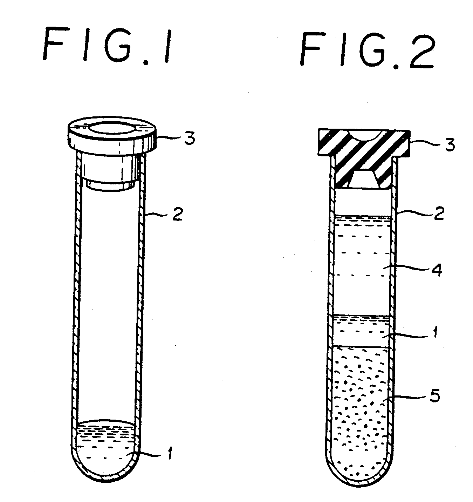

From the blood separating agent 1 obtained as described above, a blood separating tube is obtained by pouring the blood separating agent in a prescribed amount in a blind tube 2 as illustrated in Fig. 1, reducing the pressure inside the tube to a value on the order of 8 to 66,66 kPa (60 to 500 torr), preferably 13,3 to 40 kPa (100 to 300 torr),and tightly sealing the agent with a gas-impermeable stopper (3) such as, for example, a butyl rubber stopper. The materials which are usable for the tube include glass and plastics such as methacryl resin and polyethylene terephthalate, which are sparingly gas-permeable.

The separation of blood by the use of the blood separating tube is accomplished by first collecting blood in the blood separating tube, allowing the blood to stand at rest for a short while, and centrifuging the blood with a centrifugal force in the range of 700 to 1,000 g for about 10 minutes thereby allowing the blood separating agent 1 to intervene stably between the separated layers of blood serum and blood clot. In the resultant state of the tube interior, since the separating agent 1 possesses thixotropic property and an intermediate specific gravity between the blood serum 4 and the blood clot 5, it assumes its position between the blood serum 4 and the blood clot 5 as illustrated in Fig. 2, separates them, and again forms a gel.

Now, the present invention will be described more specifically below.

Referential Example 1 (Production of copolymer)

In a flask having an inner volume of 2 liters, 600 g of an $\alpha$-olefin of 12 to 14 carbon atoms (produced by Mitsubishi Kasei Corporation, and marketed under trademark designation of Dialene® 124 ) and 600 g of dimethyl maleate (hereinafter referred to as "DMM") were placed and heated under an atmosphere of nitrogen gas at 140°C, then 24.2 g of di-t-butyl peroxide (hereinafter referred to as "DTBPO") was gradually added thereto over a period of 6 hours with the temperature kept constant, and the resultant mixture was left aging at the same temperature for 4 hours.

After the reaction was completed, the reaction mixture was heat-treated at 170°C under a vacuum of 1 to 5 mmHg to expel a volatile component and an unreacted monomer by distillation, to obtain 1,140 g (95% in yield) of an $\alpha$-olefin-dimethyl maleate copolymer.

The copolymer thus obtained was found to exhibit a viscosity of 168 Pa.s (168,000 cP) and a specific gravity , $d_4^{25}$ , of 1.030 at 25°C. The compositional molar ratio of the components, dimethyl maleate/$\alpha$-olefin, of the copolymer was 1.32.

Referential Examples 2 to 4 (Production of copolymer)

Copolymers were obtained by following the procedure of Referential Example 1, except that $\alpha$-olefins and maleic diesters shown in Table 1 were used instead and the reaction conditions shown in Table 1 were employed instead.

The attributes of the produced copolymers are additionally shown in Table 1.

Table 1 (α-Olefin-maleic diester copolymer)

| | | Referential Example 1 | Referential Example 2 | Referential Example 3 | Referential Example 4 |
|---|---|---|---|---|---|
| Reaction Condition | α-Olefin (number of Carbon) | $C_{12-14}$ | $C_{12-14}$ | $C_{12-14}$ | $C_{12-14}$ |
| | Maleic diester | DMM | DMM | DMM | DMM |
| | Maleic diester/α-Olefin (adding amount, g) | 600/600 | 606/600 | 620/600 | 625/600 |
| | Adding amount of DTBPO (g) | 24.2 | 24.2 | 24.2 | 24.2 |
| | Time of addition | 6 | 9 | 2 | 1.5 |
| | Aging time | 4 | 5 | 1.25 | 1 |
| | Polymerization temp. (°C) | 140 | 135 | 160 | 180 |
| Attributes of blood separating agent | Maleic diester/α-Olefin in copolymer (molar ratio) | 1.32 | 1.33 | 1.39 | 1.39 |
| | Viscosity $Pa \cdot s$ (cP) at 25°C | 168 (168,000) | 276 (276,000) | 65 (65,000) | 20.9 (20,900) |
| | Specific gravity $d_4^{25}$ | 1,030 | 1.033 | 1.032 | 1.034 |

Examples 1 and 2 and Comparative Example 1 to 5 (Production of blood separating agent)

Gel-like blood separating agents 1 to 7 exhibiting thixotropic property were obtained by placing the copolymers (separated layers forming materials) obtained in Referential Examples 1 to 4 and dibenzylidene sorbitol (organic gelling agent) in varying compositional ratios indicated in Table 2 in four-necked flasks, heating them to a temperature in the range of 170° to 180°C, stirring them for two hours for solution of the organic gelling agent, and then cooling the stirred mixtures. The attributes of these blood separating agents are additionally shown in Table 2.

The viscosity of a given blood separating agent was determined as follows. A given gel-like blood separating agent, within the period of the 10th to 30th days of completion of the gelation, was dispensed in an amount of 40 to 50 ml approximately in centrifugal sedimentation tubes (50-ml grade, Falcon® 2070), immediately subjected to centrifugal separation (1670 g, 10 minutes), then left standing at 25°C for 24 hours, and tested for viscosity with a Vismetron viscosimeter (produced by Shibaura System K.K. and marketed under product code of "VG-H1") under the following conditions.

[Conditions of measurement]

| Rotor: | No. 7 |
|---|---|
| Revolution number: | 2 rpm |
| Measuring time: | 15 minutes after start of rotation. |
| Temperature: | 25°C |

Table 2 (Blood separating agent)

| | | Example 1 (Separating agent 1) | Example 2 (Separating agent 2) | Comparative Example 1 (Separating agent 3) | Comparative Example 2 (Separating agent 4) | Comparative Example 3 (Separating agent 5) | Comparative Example 4 (Separating agent 6) | Comparative Example 5 (Separating agent 7) |
|---|---|---|---|---|---|---|---|---|
| Composition | Copolymer (Referential Example 1) | 100 | 100 | | | | | |
| | Copolymer (Referential Example 2) | | | 100 | | | | |
| | Copolymer (Referential Example 3) | | | | 100 | 100 | | |
| | Copolymer (Referential Example 4) | | | | | | 100 | 100 |
| | Dibenzylidene sorbitol | 0.2 | 0.25 | 0.25 | 0.2 | 0.25 | 0.2 | 0.25 |
| Attributes of blood separating agent | Viscosity $Pa.s(cP)$ | 508 (508,000) | 549 (549,000) | 702 (702,000) | 270 (270,000) | 296 (296,000) | 128 (128,000) | 229, (229,000) |
| | Specific Density $d_4^{2\Gamma}$ | 1.030 | 1.030 | 1.033 | 1.032 | 1.032 | 1.034 | 1.034 |

Test Examples 1 to 8

The blood separating agents 1 to 7 obtained respectively in Example 1 and 2 and Comparative Examples 1 to 5 were tested for stability of storage and blood separating capacity by the following

8

procedures.

(1) Stability of storage:

This property was determined by pouring a given blood separating agent in a blood separating tube made of glass, keeping the tube in an upright position at 25°C for a prescribed time, holding the tube horizontally on its side under a varying temperature condition for 10 days, and measuring the length of the flow of the agent at the end of the 10 days standing. The results are shown in Table 3.

(2) Blood separating capacity:

1) Remain of blood corpuscles on supernatant surface of separating agent:

This property was determined by collecting whole blood from a human subject in a blood separating tube containing a prescribed amount of a given blood separating agent, allowing the blood to coagulate thoroughly, subjecting the blood in the tube to centrifugal separation thereby effecting separation between a blood serum component and a blood clot component by means of the blood separating agent, and measuring the amount of blood corpuscles remaining on the supernatant surface of the separating agent. The results are shown in Table 4.

2) Change of potassium concentration in blood serum with the passage of time

This property was determined by collecting whole blood from a human subject in a blood separating tube containing a prescribed amount of a given blood separating agent, subjecting the blood in the tube to centrifugal separation thereby obtaining blood serum as a supernatant layer component of the blood separating agent, holding the separating tube in storage, and measuring the potassium concentration in the blood serum at intervals during the course of the storage thereby finding the change of potassium concentration in the blood serum with the passage of time. The results are shown in Table 5.

Table 3 (Stability of storage-length of flow of separating agent) (unit: mm)

| | Inner diameter of tube (mm) | Amount of separating agent poured (ml) | Time of upright of standing (hr) | Temperature of horizontal standing (°C) | Separating agent 1 | Separating agent 2 | Separating agent 3 | Separating agent 4 | Separating agent 5 | Separating agent 6 | Separating agent 7 |
|---|---|---|---|---|---|---|---|---|---|---|---|
| Test Example 1 | 10.8 | 1.1 | 18 | 20 | 1.5 | 1.6 | 0.1 | 1.0 | 0.2 | 2.2 | 1.9 |
| Test Example 2 | 10.8 | 1.1 | 216 | 50 | 1.8 | 1.4 | 0.5 | 10.3 | 4.5 | 13.7 | 4.4 |
| Test Example 3 | 13.6 | 1.7 | 18 | 20 | 1.8 | 1.0 | 0.2 | 1.1 | 0.2 | 2.6 | 2.0 |
| Test Example 4 | 13.6 | 1.7 | 216 | 50 | 2.2 | 1.9 | 0.7 | 10.9 | 5.1 | 15.7 | 5.2 |

Table 4 (Remain of blood corpuscles on supernatant surface of separating agent)
[Conditions] In a blood separating tube made of glass and having an inside diameter of 13.6 mm, 1.7 ml of a given blood separating agent was placed, 9 ml of whole blood from the human body was collected, the blood was coagulated thoroughly and subjected to centrifugal separation at 1,200 g for 10 minutes. The amount of blood corpuscles remaining on the supernatant surface of the separating agent was measured.

1. Measurement performed 10 days after the placement

| | Separating agent 1 | Separating agent 2 | Separating agent 3 | Separating agent 4 | Separating agent 5 | Separating agent 6 | Separating agent 7 |
|---|---|---|---|---|---|---|---|
| Test Example 5 | A | A | B | A | B | B | A |

2. Measurement performed 60 days after the placement

| | Separating agent 1 | Separating agent 2 | Separating agent 3 | Separating agent 4 | Separating agent 5 | Separating agent 6 | Separating agent 7 |
|---|---|---|---|---|---|---|---|
| Test Example 6 | A | A | C | D | D | D | C |

[Symbols for rating]

$$\text{Small} \; \frac{\text{Remaining Amount of blood corpuscles}}{A \qquad B \qquad C \qquad D} \; \text{Large}$$

EP 0 375 566 B1

Table 5 (Change of potassium concentration in blood serum with the passage of time)

[Conditions] In a blood separating tube made of glass and having an inside diameter of 13.6 mm, 1.7 ml of a given blood separating agent was placed, 9 ml of whole blood from the human body was collected, the blood was coagulated thoroughly and subjected to centrifugal separation at 1,200 g for 10 minutes, and the resultant specimen was preserved at 5°C for 14 days. During this storage, the potassium concentration in the blood serum was measured at intervals along the course of time.

1. Measurement performed 10 days after the placement

(unit: meq/1)

| | Separating agent 1 | Separating agent 2 | Separating agent 3 | Separating agent 4 | Separating agent 5 | Separating agent 6 | Separating agent 7 |
|---|---|---|---|---|---|---|---|
| Test Example 7 | 0.34 | 0.32 | 0.56 | 0.63 | 0.65 | 0.35 | 0.40 |

2. Measurement performed 60 days after the placement

(unit: meq/1)

| | Separating agent 1 | Separating agent 2 | Separating agent 3 | Separating agent 4 | Separating agent 5 | Separating agent 6 | Separating agent 7 |
|---|---|---|---|---|---|---|---|
| Test Example 8 | 0.48 | 0.45 | 0.69 | 0.77 | 0.80 | 0.71 | 0.68 |

## Claims

1. A blood separating agent comprising (A) a separated layers forming material formed of a copolymer of an $\alpha$-olefin of 6 to 20 carbon atoms with a maleic diester in which the number of carbons of the alkyl group is in the range of 1 to 14, and possessing a compositional ratio of 1.0 to 1.4 mols of the maleic diester per 1.0 mol of the $\alpha$-olefin in the copolymer and a viscosity in the range of 100 to 250 Pa.s (100,000 to 250,000 cP) at 25°C and (B) 0.12 to 0.30 part by weight, based on 100 parts by weight of said separated layers forming material, of an organic gelling agent formed of a condensate of sorbitol and benzaldehyde, and the blood separating agent possessing a specific gravity in the range of 1.028 to 1.050 (at 25°C).

12

**2.** A blood separating agent according to claim 1, wherein the number of carbon atoms of said α-olefin is in the range of 10 to 16.

**3.** A blood separating agent according to claim 1, wherein said condensate of a sorbitol and benzaldehyde is at least one member selected from the group consisting of dibenzylidene sorbitol, tribenzylidene sorbitol, and methyl-substituted dibenzylidene sorbitol.

**4.** A blood separating agent according to claim 1, wherein said maleic diester is a dialkyl ester of maleic acid and the number of carbon atoms of the alkyl group thereof is in the range of 1 to 8.

**5.** A blood separating agent according to claim 1, wherein the amount of said organic gelling agent is in the range of 0.14 to 0.30 part by weight, based on 100 parts by weight of said separated layers forming material.

**6.** A blood separating agent according to claim 1, wherein the viscosity of said blood separating agent is in the range of 300 to 700 Pa.s (300,000 to 700,000 cP) (at 25°C).

**7.** A blood separating agent according to claim 1, wherein the viscosity of said separated layers forming material is 120 to 200 Pa.s (120,000 to 200,000 cP) (at 25°C).

**8.** A blood separating tube having encapsulated in a blind tube a blood separating agent according to claim 1.

**9.** A blood separating tube according to claim 8, wherein said blind tube is a tube made of glass or plastics and provided with a tightly sealing stopper.

**10.** A blood separating tube according to claim 9, wherein said blind tube is retained in a vacuum state.

**11.** A blood separating tube according to claim 8, wherein the number of carbon atoms of said α-olefin is in the range of 10 to 16.

**12.** A blood separating tube according to claim 8, wherein said condensate of a sorbitol and benzaldehyde is at least one member selected from the group consisting of dibenzylidene sorbitol, tribenzylidene sorbitol, and methyl-substituted dibenzylidene sorbitol.

**13.** A blood separating tube according to claim 8, wherein said maleic diester is a dialkyl ester of maleic acid and the number of carbon atoms of the alkyl group thereof is in the range of 1 to 8.

**14.** A blood separating tube according to claim 8, wherein the amount of said organic gelling agent is in the range of 0.14 to 0.30 part by weight, based on 100 parts by weight of said separated layers forming material.

**15.** A blood separating tube according to claim 8, wherein the viscosity of said blood separating agent is in the range of 300 to 700 Pa.s (300,000 to 700,000 cP) (at 25°C).

**16.** A blood separating tube according to claim 8, wherein the viscosity of said separated layers forming material is 120 to 200 Pa.s (120,000 to 200,000 cP) (at 25°C).

**Patentansprüche**

**1.** Bluttrennmittel, umfassend (A) ein getrennte Schichten bildendes Material aus einem Copolymeren eines α-Olefins mit 6 bis 20 Kohlenstoffatomen und einem Maleinsäurediester, bei dem die Anzahl der Kohlenstoffe in der Alkylgruppe im Bereich von 1 bis 14 liegt, wobei das (Zusammensetzungs-)Verhältnis in dem Copolymeren 1,0 bis 1,4 Mol(e) des Maleinsäurediesters pro 1,0 Mol des α-Olefins beträgt und das Copolymere eine Viskosität im Bereich von 100 bis 250 Pa•s (100 000 bis 250 000 cP) bei 25°C aufweist, und (B) 0,12 bis 0,30 Gewichtsteil, bezogen auf 100 Gewichtsteile des die getrennten Schichten bildenden Materials, eines aus einem Kondensat aus Sorbit und Benzaldehyd gebildeten organischen Geliermittels, wobei das Bluttrennmittel ein spezifisches Gewicht (bei 25°C) im Bereich von 1,028 bis 1,050 aufweist.

2. Bluttrennmittel nach Anpruch 1, wobei die Anzahl an Kohlenstoffatomen in dem $\alpha$-Olefin im Bereich von 10 bis 16 liegt.

3. Bluttrennmittel nach Anspruch 1, wobei das Kondensat aus einem Sorbit und Benzaldehyd aus mindestens einer Komponente aus der Gruppe Dibenzylidensorbit, Tribenzylidensorbit und methylsubstituierter Dibenzylidensorbit besteht.

4. Bluttrennmittel nach Anspruch 1, wobei es sich bei dem Maleinsäurediester um einen Dialkylester der Maleinsäure handelt und die Anzahl der Kohlenstoffatome seiner Alkylgruppe im Bereich von 1 bis 8 liegt.

5. Bluttrennmittel nach Anspruch 1, wobei die Menge an dem organischen Geliermittel im Bereich von 0,14 bis 0,30 Gewichtsteil, bezogen auf 100 Gewichtsteile des getrennte Schichten bildenden Materials, liegt.

6. Bluttrennmittel nach Anspruch 1, wobei die Viskosität des Bluttrennmittels im Bereich von 300 bis 700 Pa•s (300 000 bis 700 000 cP) (bei 25°C) beträgt.

7. Bluttrennmittel nach Anspruch 1, wobei die Viskosität des getrennte Schichten bildenden Materials 120 bis 200 Pa•s (120 000 bis 200 000 cP) (bei 25°C) beträgt.

8. Bluttrennrohr mit einem in einem Blindrohr eingekapselten Bluttrennmittel nach Anspruch 1.

9. Bluttrennrohr nach Anspruch 8, wobei das Blindrohr aus einem aus Glas oder Kunststoffen gebildeten Rohr besteht und mit einem dicht verschließenden Stopfen versehen ist.

10. Bluttrennrohr nach Anspruch 9, wobei das Blindrohr unter Vakuum gehalten ist.

11. Bluttrennrohr nach Anspruch 8, wobei die Anzahl der Kohlenstoffatome des $\alpha$-Olefins im Bereich von 10 bis 16 liegt.

12. Bluttrennrohr nach Anspruch 8, wobei das Kondensat aus einem Sorbit und Benzaldehyd aus mindestens einer Komponente aus der Gruppe Dibenzylidensorbit, Tribenzylidensorbit und methylsubstituierter Dibenzylidensorbit besteht.

13. Bluttrennrohr nach Anspruch 8, wobei es sich bei dem Maleinsäurediester um einen Dialkylester der Maleinsäure handelt und die Anzahl der Kohlenstoffatome seiner Alkylgruppe im Bereich von 1 bis 8 liegt.

14. Bluttrennrohr nach Anspruch 8, wobei die Menge an dem organischen Geliermittel im Bereich von 0,14 bis 0,30 Gewichtsteil, bezogen auf 100 Gewichtsteile des getrennte Schichten bildenden Materials, liegt.

15. Bluttrennrohr nach Anspruch 8, wobei die Viskosität des Bluttrennmittels im Bereich von 300 bis 700 Pa•s (300 000 bis 700 000 cP) (bei 25°C) beträgt.

16. Bluttrennrohr nach Anspruch 8, wobei die Viskosität des getrennte Schichten bildenden Materials 120 bis 200 Pa•s (120 000 bis 200 000 cP) (bei 25°C) beträgt.

**Revendications**

1. Agent de séparation du sang comprenant (A) une matière formant des couches séparées formée d'un copolymère d'une $\alpha$-oléfine en $C_6$-$C_{20}$ avec un diester maléique dont les groupes alkyles sont en $C_1$-$C_{14}$ et présentant un rapport de 1,0 à 1,4 mol du diester maléique pour 1,0 mol de l'$\alpha$-oléfine dans le copolymère et une viscosité dans la gamme de 100 à 250 Pa.s (100 000 à 250 000 cP) à 25°C et (B) 0,12 à 0,30 partie en poids d'un agent gélifiant organique formé d'un condensat de sorbitol et de benzaldéhyde, pour 100 parties en poids de ladite matière formant des couches séparées et l'agent de séparation du sang possédant un poids spécifique dans la gamme de 1,028 à 1,050 à 25°C.

2. Agent de séparation du sang selon la revendication 1, dans lequel ladite $\alpha$-oléfine a une chaîne en $C_{10}$-$C_{16}$.

3. Agent de séparation du sang selon la revendication 1, dans lequel ledit condensat d'un sorbitol et de benzaldéhyde comprend au moins un composé choisi parmi le dibenzylidène-sorbitol, le tribenzylidène-sorbitol et le dibenzylidène-sorbitol méthylsubstitué.

4. Agent de séparation du sang selon la revendication 1, dans lequel ledit diester maléique est un ester de dialkyle comprenant des groupes alkyles en $C_1$-$C_8$.

5. Agent de séparation du sang selon la revendication 1, dans lequel la quantité dudit agent gélifiant organique est dans la gamme de 0,14 à 0,30 partie en poids pour 100 parties en poids de ladite matière formant des couches séparées.

6. Agent de séparation du sang selon la revendication 1, ayant une viscosité dans la gamme de 300 à 700 Pa.s (300 000 à 700 000 cP) à 25°C.

7. Agent de séparation du sang selon la revendication 1, dans lequel la viscosité de ladite matière formant des couches séparées est de 120 à 200 Pa.s (120 000 à 200 000 cP) à 25°C.

8. Tube de séparation du sang contenant un agent de séparation du sang selon la revendication 1 encapsulé dans un tube aveugle.

9. Tube de séparation du sang selon la revendication 8, dans lequel ledit tube aveugle est un tube en verre ou en matière plastique muni d'un bouchon fermant hermétiquement.

10. Tube de séparation du sang selon la revendication 9, dans lequel ledit tube aveugle est maintenu sous vide.

11. Tube de séparation selon la revendication 8, dans lequel ladite $\alpha$-oléfine a une chaîne en $C_{10}$-$C_{16}$.

12. Tube de séparation selon la revendication 8, dans lequel ledit condensat d'un sorbitol et de benzaldéhyde comprend au moins un composé choisi parmi le dibenzylidène-sorbitol, le tribenzylidène-sorbitol et le dibenzylidène-sorbitol méthylsubstitué.

13. Tube de séparation selon la revendication 8, dans lequel ledit diester maléique est un ester de dialkyle comprenant des groupes alkyles en $C_1$-$C_8$.

14. Tube de séparation selon la revendication 8, dans lequel la quantité dudit agent gélifiant organique est dans la gamme de 0,14 à 0,30 partie en poids pour 100 parties en poids de ladite matière formant des couches séparées.

15. Tube de séparation selon la revendication 8, ayant une viscosité dans la gamme de 300 à 700 Pa.s (300 000 à 700 000 cP) à 25°C.

16. Tube de séparation selon la revendication 8, dans lequel la viscosité de ladite matière formant des couches séparées est de 120 à 200 Pa.s (120 000 à 200 000 cP) à 25°C.

# FIG.1

# FIG.2